# EUROPEAN PATENT SPECIFICATION

(11) **EP 3 783 365 B1**
(45) Date of publication and mention of the grant of the patent: **07.06.2023**
(21) Application number: 19201755.6
(22) Date of filing: 07.10.2019
(51) Int. Cl.: G01N 33/94, G01N 33/543, G01N 33/58

(54) **IMMUNOCHROMATOGRAPHIC TEST STRIP FOR DETECTING DIGOXIN AND USE THEREOF**
IMMUNOCHROMATOGRAPHISCHER TESTSTREIFEN ZUM NACHWEIS VON DIGOXIN UND DESSEN VERWENDUNG
BANDE D'ESSAI IMMUNOCHROMATOGRAPHIQUE POUR DÉTECTER LA DIGOXINE ET SON UTILISATION

(30) Priority: 23.08.2019 CN 201910782799
(43) Date of publication of application: 24.02.2021
(73) Proprietor: Beijing Diagreat Biotechnologies Co., Ltd., Beijing 101111 (CN)
(72) Inventor: ZHOU, Jianping, BEIJING, Beijing 101111 (CN); ZHOU, Yujun, BEIJING, Beijing 101111 (CN); XU, Xiuli, BEIJING, Beijing 101111 (CN)
(74) Representative: Bottero, Carlo

(56) References cited:
- WO-A1-2017/132614
- CN-A- 109 061 134
- CN-A- 109 061 152
- CN-A- 109 813 691
- US-A- 5 248 619

## Description

### TECHNICAL FIELD

The present invention relates to the field of immunodetection technology, and in particular to an immunochromatographic test strip for detecting digoxin and a use thereof.

### BACKGROUND

Digoxin is a medium-effect cardiac glycoside drug. During treatment, its effect on the heart is presented as a positive inotropic effect, which slows a heart rate and inhibits the cardiac conduction. It is suitable for a low-output congestive heart-failure, atrial fibrillation, atrial flutter, and paroxysmal supraventricular tachycardia. Among digitalis drugs, digoxin is excreted faster and less accumulated, and oral absorption of it is incomplete and irregular. In the "2018 Guidelines for the Diagnosis and Treatment of Heart Failure in China", it is recommended that adverse reactions such as digoxin toxicity should be strictly monitored and the drug concentration should be maintained at 0.5-0.9 ng/mL. 0.8-2 ng/ml is generally used as an effective therapeutic concentration range. There is a huge difference in the pharmacokinetics of digoxin among individuals, and both in vitro and in vivo factors influence the pharmacokinetics of digoxin. Therefore, monitoring the drug concentration in a patient treated with digoxin is of great significance for the treatment clinical safety, rational drug use and individualized medication.

With the initiative of precision medicine and personalized medicine, detection of various drug concentrations is receiving more and more attention. At present, the commonly used methods for detecting digoxin are mainly mass spectrometry, high-performance liquid chromatography, immunoturbidimetry, chemiluminescence, and enzyme multiplied immunization, where the mass spectrometry is a method in which electric and magnetic fields are used to separate moving ions according to their mass-to-charge ratios, and then a detection is conducted, the detection being conducted using a mass spectrometer. The high performance liquid chromatography uses a liquid as a mobile phase and adopts a high pressure infusion system to pump mobile phases, such as a single solvent having different polarities or mixed solvents of different proportions, a buffer solution into a chromatographic column filled with a stationary phase. After separated, the components in the column enter a detector for detection, thus realizing analysis of the sample. The analysis instrument is a high performance liquid chromatograph instrument. The results of chromatography and mass spectrometry are accurate, but sample preparation is required before sample loading, the detection time is long, and only one sample can be loaded at a time, so that the high-throughput requirement cannot be achieved, and the cost of the detection instrument as used is high, which imparts a certain limitation on clinical promotion. The immunoturbidimetry is a method that is mainly used by Roche for detection, which requires a matched high-cost biochemical analyzer and refrigerated storage of the reagents, being not able to meet the increasing clinical detection demands and being not able to achieve detection for a single person at any time. The chemiluminescence is a method that is mainly used by Abbott Diagnostics and Roche for detection. For this method, it is necessary to provide a high-cost chemiluminescence instrument, the reagent needs to be stored under refrigeration, and the package is 100 servings/kit. Some small hospitals cannot have such a high sample size, resulting in waste of reagents or a too long time of bottle opening. There are also applications for patents of a chemiluminescence method in China (Zecen Biotech Co., Ltd. (Taizhou), application number 201711217116.0). The main representative manufacturer that uses the enzyme multiplied immunization method to detect digoxin is Siemens. By using the immunization method, multiple samples can be simultaneously determined on an automatic biochemical analyzer to achieve high-throughput rapid determination. The method requires the use of an automatic biochemical analyzer, and the storage temperature is 2-8°C, which requires cold-chain transportation, cannot be made into a single-serving package, and cannot achieve the purpose of timely monitoring in a medication department.

### SUMMARY

In view of the above, an objective of the present invention is to provide an immunochromatographic test strip for detecting digoxin and a use thereof. The immunochromatographic test strip provided by the present invention has a wide sample type, excellent stability and high sensitivity.

In order to achieve the foregoing invention objective, the present invention provides the following technical solutions:
The present invention provides an immunochromatographic test strip for detecting digoxin, which includes a bottom plate, and a sample pad, a glass fiber mat, a nitrocellulose membrane and a water absorbing paper which are sequentially overlapped on the bottom plate, where the glass fiber mat is sprayed with a digoxin-specific antibody-fluorescent microsphere complex; and the nitrocellulose membrane is sequentially provided with a detection line coated with digoxin protein conjugate and a quality control line coated with a secondary antibody;
the digoxin-specific antibody-fluorescent microsphere complex is obtained by conjugation between a digoxin monoclonal antibody or a digoxin polyclonal antibody and a fluorescent microsphere;
the digoxin protein conjugate includes a digoxin-conjugated rabbit albumin or a digoxin-conjugated rabbit ovalbumin; the concentration of the digoxin-conjugated rabbit albumin is 1 mg/mL; the concentration of the digoxin-conjugated rabbit ovalbumin is 3 mg/mL. The mass percentage concentration of the digoxin-specific antibody-fluorescent microsphere complex is 0.01-0.1%; and the spraying amount of the digoxin-specific antibody-fluorescent microsphere complex on the glass fiber mat is 1-10 µl/cm.

The spraying amount of the digoxin protein conjugate on the nitrocellulose membrane is 1-5 µl/cm.

The secondary antibody includes a goat anti-mouse IgG antibody.

Preferably, the concentration of the goat anti-mouse IgG antibody is 1-3 mg/mL.

The sample pad includes a glass fiber, a polyester film, a cellulose filter paper, and a nonwoven fabric.

The present invention further provides a use of the immunochromatographic test strip described in the aforementioned technical solution for detecting digoxin content in a sample.

Preferably, the detecting the digoxin content in the sample includes the steps of:
1) adding a sample to be tested dropwise onto a sample pad for chromatography;
2) reading fluorescence signal values, i.e., a T value and a C value, for the detection line and the quality control line of the immunochromatographic test strip after the chromatography; and
3) substituting the fluorescence signal values T/C into a standard linear regression equation *y* = *ax*³ + *bx²* + *cx* + *d* to calculate the digoxin content in the sample to be tested.

Preferably, the test sample includes serum, plasma and whole blood.

Compared with the prior art, the immunochromatographic test strip for detecting digoxin and the use thereof provided by the present invention have the following advantages:
(1) as compared with mass spectrometry and high-performance liquid chromatography, the immunochromatographic test strip provided by the present invention has a low detection cost, avoids a complicated pre-processing process, allows for quick detection, can realize high-throughput detection, is packed in a single serving, has excellent stability and high repeatability; and
(2) as compared with turbidimetry and chemiluminescence, the immunochromatographic test strip provided by the present invention has a low detection cost, can be stored at room temperature, is packaged in a single serving, has the sample types of serum, plasma or whole blood, has excellent stability and high sensitivity.

### BRIEF DESCRIPTION OF THE DRAWING

FIG. 1 is a diagram showing a calibration curve of digoxin; and
FIG. 2 is a diagram showing the correlation of a digoxin detection reagent.

### DETAILED DESCRIPTION

The present invention provides an immunochromatographic test strip for detecting digoxin, which includes a bottom plate, and a sample pad, a glass fiber mat, a nitrocellulose membrane and a water absorbing paper which are sequentially overlapped on the bottom plate; the glass fiber mat is sprayed with a digoxin-specific antibody-fluorescent microsphere complex; and the nitrocellulose membrane is sequentially provided with a detection line coated with digoxin protein conjugate and a quality control line coated with a secondary antibody;
the digoxin-specific antibody-fluorescent microsphere complex is obtained by conjugation between a digoxin monoclonal antibody or a digoxin polyclonal antibody and a fluorescent microsphere; and
the digoxin protein conjugate includes a digoxin-conjugated rabbit albumin or a digoxin-conjugated rabbit ovalbumin.

The present invention has no specific limitation on the type of the bottom plate, and a bottom plate used by a conventional test strip can be used.

The present invention has no specific limitation on the material of the sample pad, and the sample pad includes a glass fiber, a polyester film, a cellulose filter paper, and a nonwoven fabric. In the present invention, the sample pad is preferably subjected to an immersion treatment, and the chemical for the immersion treatment is preferably a Triton X-100 nonionic surfactant.

The present invention has no specific limitation on the source of the glass fiber mat, and a commercially available product of a conventionally-prepared test strip can be used. In the present invention, the glass fiber mat acts as a conjugate pad. The glass fiber mat of the present invention is preferably sprayed with a digoxin-specific antibody-fluorescent microsphere complex thereon; the digoxin-specific antibody-fluorescent microsphere complex is preferably obtained by conjugation between a digoxin monoclonal antibody or a digoxin polyclonal antibody and a fluorescent microsphere; the mass percentage concentration of the digoxin-specific antibody-fluorescent microsphere complex is 0.01-0.1%; and the spraying amount of the digoxin-specific antibody-fluorescent microsphere complex on the glass fiber mat is 1-10 µl/cm, preferably 3-7 µl/cm, and more preferably 5 µl/cm.

The present invention has no specific limitation on the source of the nitrocellulose membrane, and a commercially available product of a conventionally-prepared test strip can be used. In the present invention, the nitrocellulose membrane is sequentially provided with a detection line coated with digoxin protein conjugate and a quality control line coated with a secondary antibody; the digoxin protein conjugate includes a digoxin-conjugated rabbit albumin or a digoxin-conjugated rabbit ovalbumin; the concentration of the digoxin-conjugated rabbit albumin is 1 mg/mL and the concentration of the digoxin-conjugated rabbit ovalbumin is 3 mg/mL; the spraying amount of the digoxin protein conjugate on the nitrocellulose membrane is 1-5 µl/cm, preferably 2-4 µl/cm, and more preferably 5 µl/cm; the secondary antibody includes a goat anti-mouse IgG antibody; and the concentration of the goat anti-mouse IgG antibody is preferably 1-3 mg/mL.

The present invention also provides the use of the immunochromatographic test strip described in the aforementioned technical solution for detecting digoxin content in a sample. It preferably includes the steps of 1) adding a sample to be tested dropwise onto a sample pad for chromatography; 2) reading fluorescence signal values, i.e., a T value and a C value, for the detection line and the quality control line of the immunochromatographic test strip after the chromatography; and 3) substituting the fluorescence signal values T/C into a standard linear regression equation *y* = *ax*³ + *bx*² + *cx* + *d* to calculate the digoxin content in the sample to be tested.

In the present invention, the test sample preferably includes serum, plasma, and whole blood.

The following clearly and completely describes the technical solutions in the present invention with reference to the embodiments of the present invention. Apparently, the described embodiments are merely a part rather than all of the embodiments of the present invention.

### Embodiment 1

### Preparation of Immunochromatographic Test Strip:

(1) a labeling process: A. fluorescent microsphere labeling: the fluorescent microspheres were purchased from common fluorescent microspheres on the market at a particle size of 80 nm, 1% fluorescent microspheres, 10 mg/ml of EDC, and 100 µg/ml of a digoxin monoclonal antibody were added, mixed well for conjugation for 3 h, centrifuged with the supernatant being removed, and then added with 1% BSA for blocking for 1 h; and B. colloidal gold labeling: colloidal gold was a colloidal gold solution with a particle size of 10 nm as prepared by a general-purpose trisodium citrate reduction method, 100 µg/ml of the digoxin monoclonal antibody was added, mixed well for conjugation for 3 h, centrifuged with the supernatant being removed, and then added with 1% BSA for blocking for 1 h;
(2) a gold spraying process: the prepared labeled conjugate was centrifuged, and resuspended in a gold spraying buffer to a concentration of 0.2% for gold spraying, where A. the resuspension buffer was: a 50 mM buffer (tris, phosphoric acid, glycine, HEPES, PH = 6.5), 1 g/L of sucrose; 3 g/L of polyethylene glycol 6000; 0.1 g/L of glycine; 0.05 g/L of arginine; and 3 g/L of mannitol; B. it was necessary to pre-treat a gold cushion before gold spraying, where the treatment buffer was, a 50 mM buffer (tris, phosphoric acid, glycine, HEPES, PH = 6.5, being consistent with the gold spraying buffer), 0.1% txiton-100, and 3 g/L of mannitol; C. the gold spraying equipment as used is a gold spray membrane scribing meter; and D. after the gold spraying was completed, the product was baked in a 30°C air drying oven for drying for 6 h;
(3) a membrane scribing process: A. the digoxin protein conjugate was a digoxin-conjugated rabbit albumin, at a concentration of 1 mg/ml; B. an independent control line was goat anti-mouse IgG, at a concentration of 1 mg/ml; C. membrane scribing is conducted for the conjugate and the quality control line respectively on a T line position and a C line position of the nitrocellulose membrane; and D. after the membrane scribing was completed, the product was baked in a 30°C air drying oven for drying for 4 h;
(4) a sample pad treatment process: the sample pad was soaked in the 0.1% txiton-100 for 2 h; for promoting sample release;
(5) a board pasting process: this process was a general process, in which the sample pad, the dried gold standard pad, the dried nitrocellulose film and an absorbent paper were sequentially adhered to a bottom board; and
(6) a strip cutting and packing process: this process was a general process, in which a large reagent plate was cut, then loaded into a reagent card, added with a desiccant and sealed with an aluminum foil bag to obtain the test strip.

### Embodiment 2

Preparation of Immunochromatographic Test Strip:
(1) a labeling process: A. fluorescent microsphere labeling: the fluorescent microspheres were purchased from common fluorescent microspheres on the market at a particle size of 120 nm, 1% fluorescent microspheres, 10 mg/ml of EDC, and 500 µg/ml of a digoxin polyclonal antibody were added, mixed well for conjugation for 3 h, centrifuged with the supernatant being removed, and then added with 1% BSA for blocking for 1 h; and B. colloidal gold labeling: colloidal gold was a colloidal gold solution with a particle size of 50 nm as prepared by a general-purpose trisodium citrate reduction method, 500 µg/ml of the digoxin polyclonal antibody was added, mixed well for conjugation for 3 h, centrifuged with the supernatant being removed, and then added with 1% BSA for blocking for 1 h;
(2) a gold spraying process: the prepared labeled conjugate was centrifuged, and resuspended in a gold spraying buffer to a concentration of 0.2% for gold spraying, where A. the resuspension buffer was: a 200 mM buffer (tris, phosphoric acid, glycine, HEPES, PH = 7.8), 2g/L of sucrose; 10g/L of polyethylene glycol 6000; 1g/L of glycine; 0.5g/L of arginine; and 15g/L of mannitol; B. it was necessary to pre-treat a gold cushion before gold spraying, where the treatment buffer was, a 200 mM buffer (tris, phosphoric acid, glycine, HEPES, PH = 7.8, being consistent with the gold spraying buffer), 0.1% txiton-100, and 15g/L of mannitol; C. the gold spraying equipment as used is a gold spray membrane scribing meter; and D. after the gold spraying was completed, the product was baked in a 40°C air drying oven for drying for 10 h;
(3) a membrane scribing process: A. the digoxin protein conjugate was a digoxin-conjugated rabbit ovalbumin, at a concentration of 3mg/ml; B. an independent control line was goat anti-mouse IgG, at a concentration of 3mg/ml; C. membrane scribing was conducted for the conjugate and the quality control line respectively on a T line position and a C line position of the nitrocellulose membrane; and D. after the membrane scribing was completed, the product was baked in a 40°C air drying oven for drying for 10 h;
(4) a sample pad treatment process: the sample pad was soaked in the 0.1% txiton-100 for 2 h; for promoting sample release;
(5) a board pasting process: this process was a general process, in which the sample pad, the dried gold standard pad, the dried nitrocellulose film and an absorbent paper were sequentially adhered to a bottom board; and
(6) a strip cutting and packing process: this process was a general process, in which a large reagent plate was cut, then loaded into a reagent card, added with a desiccant and sealed with an aluminum foil bag to obtain the test strip.

### Embodiment 3

### Preparation of Immunochromatographic Reagent Strip:

(1) fluorescent microspheres with a particle size of 80 nm were used; where 1% fluorescent microspheres, 10 mg/ml of EDC, 200 µg/ml of the digoxin monoclonal antibody were added, mixed well for conjugation for 3 h, centrifuged with the supernatant being removed, and then added with 1% BSA for blocking for 1 h;
(2) the prepared labeling conjugate was centrifuged, and resuspended to 0.2% by using a resuspension buffer (a 200 mM glycine buffer, PH = 6.5), 1 g/L of sucrose; 10 g/L of polyethylene glycol 6000; 1 g/L of glycine; 0.5 g/L of arginine; 15 g/L of mannitol) , the gold cushion was soaked in a pretreatment buffer (a 200 mM glycine buffer, 0.1% txiton-100, and 15 g/L of mannitol) for 2 h, baked for 1 h; the resuspended solution was sprayed onto the gold cushion to form a membrane by using the gold spray membrane scribing meter, and after the gold spraying was completed, the product was baked in a 30°C air drying oven for drying for 10 h;
(3) The T line was a digoxin-conjugated BSA at a concentration of 1 mg/ml; the C line was a goat anti-mouse IgG at a concentration of 1 mg/ml; membrane scribing was conducted for the conjugate and the quality control line respectively on a T line position and a C line position of the nitrocellulose membrane, and after the membrane scribing was completed, the product was baked in a 40°C air drying oven for drying for 4 h;
(4) a sample pad treatment process: the sample pad was soaked in the 0.1% txiton-100 for 2 h; for promoting sample release; and
(5) The sample pad, the dried gold standard pad, the dried nitrocellulose membrane and the absorbent paper were sequentially adhered onto the bottom plate; the large reagent plate was cut, then loaded into the reagent card, added with a desiccant and sealed with an aluminum foil bag to obtain the test strip.

### Embodiment 4

### Preparation of Calibration Curve:

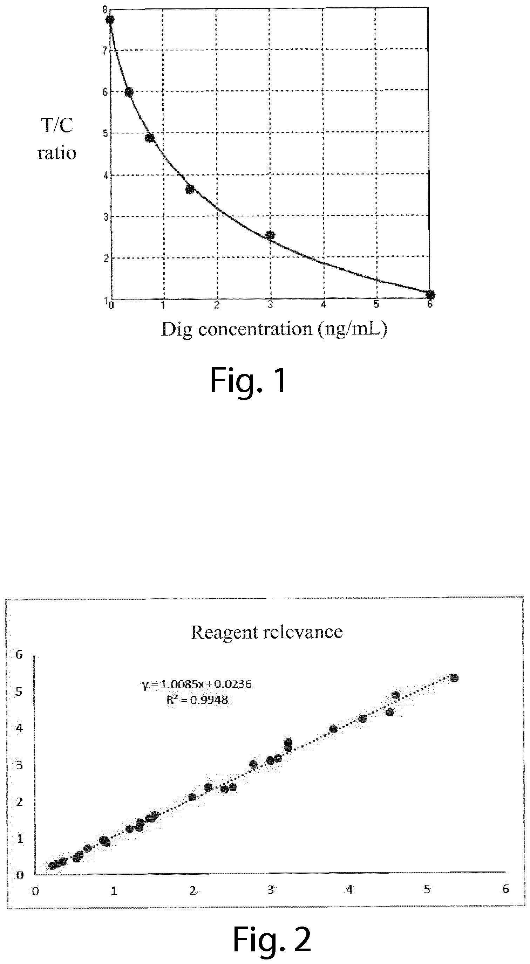

Digoxin calibrators at concentrations respectively of 0, 0.3125, 0.625, 1.25, 2.5, and 5 ng/ml were added dropwise onto the test strip of Embodiment 3, and 3 replicates were set for each concentration. After the test strip was mixed well and subjected to static chromatography for 15 min, the fluorescence signal value was read by using an immunofluorescence analyzer, and so as to calculate a T/C value and establish a calibration curve, where the X-axis is the concentration of the calibrator, and the Y-axis is the T/C value, as shown in FIG. 1.

### Detection of Sample Repeatability:

The test sample was added dropwise into a sample loading well, and 10 replicates were set for each sample. The test sample was a serum sample, and no high-value natural sample could be obtained due to the metabolism process of digoxin, so the high-value sample was obtained by adding a digoxin pure product into a clinical serum sample. The specific data is shown in Table 1:

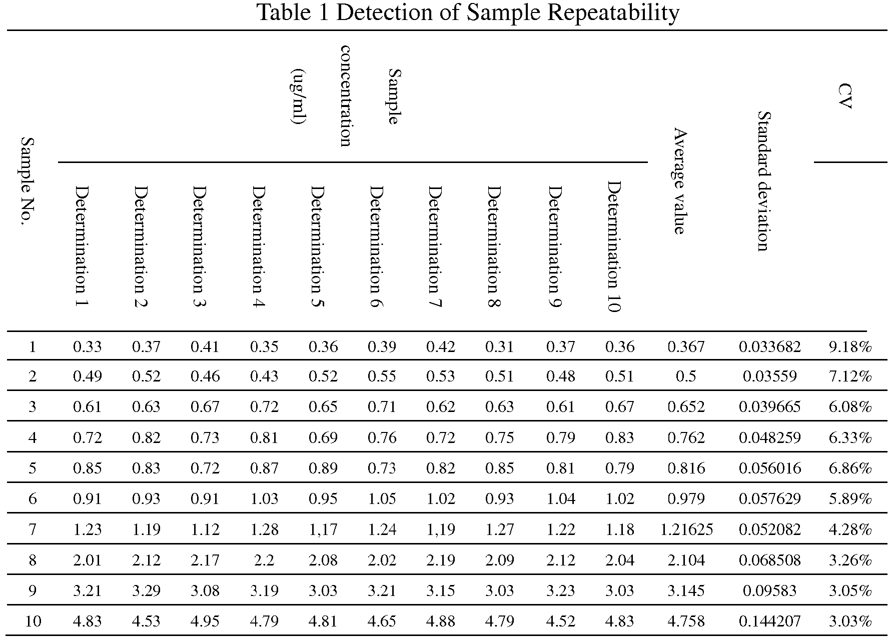

It can be seen from Table 1 that, the samples with different concentrations in the whole linear range were tested, and all of the CVs of the detection results were less than 10%, with a good repeatability, which satisfied the test requirements.

### Embodiment 5

### Detection of Anti-interference Property

The test sample was added with 50 mg/ml hemoglobin and 50 mg/dl triglyceride for routine blood detection, the values before and after the addition were tested to observe whether a conventional clinical interference substance has an effect on the determination results, and see Table 2 for the results;

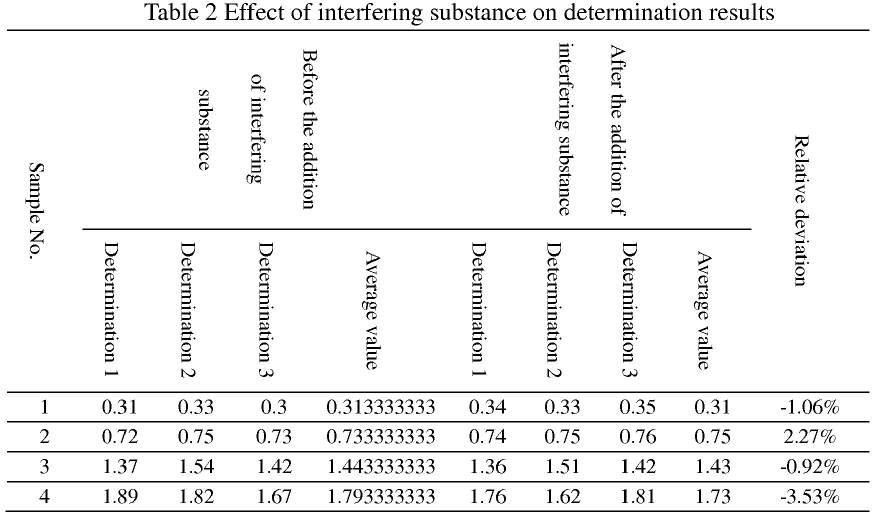

It can be seen from Table 2 that, the relative deviation before and after the addition of the interfering substance was less than 5%, which was an acceptable range.

### Embodiment 6

Comparison of whole blood sample detection results with serum sample detection results:
The whole blood sample selected in this experiment was the sample that has the same source as the serum sample. The reliability of the detection of the whole blood sample by this reagent can be determined by comparing the serum detection results and the whole blood detection results, as shown in Table 3.

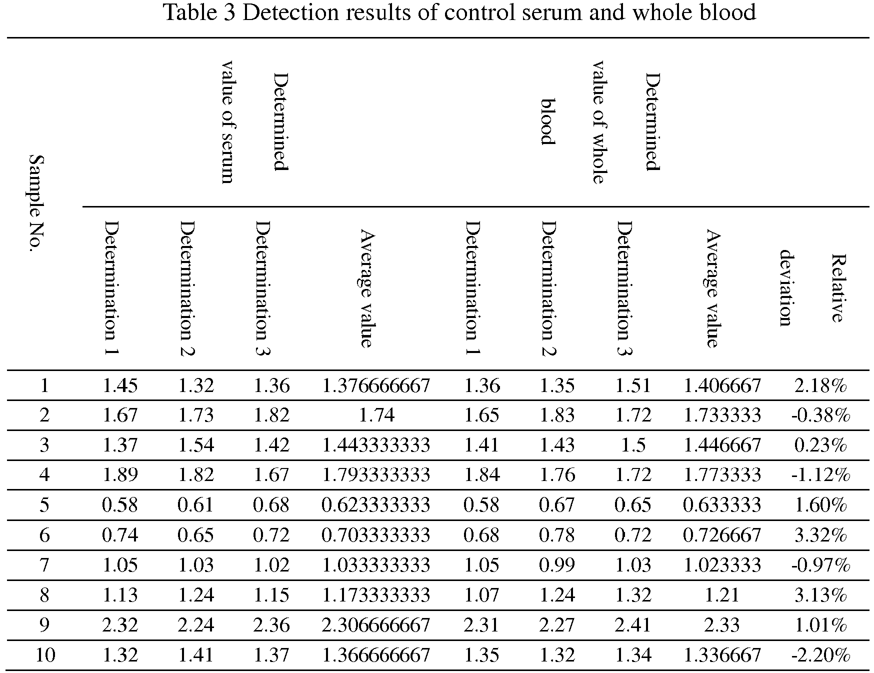

It can be seen from Table 3 that, the deviation between the whole blood sample and the serum sample was within 5%, which was acceptable.

### Comparative Embodiment 1

Comparison of stability: the shelf life of the test strip from Embodiment 3 of the present invention was 18 months when stored at room temperature 2-30°C, and the shelf life of a control reagent, i.e., a digoxin determination kit (legal luminescent microparticle immunoassay) produced by Abbott Laboratories was 18 months when stored at 2- 8°C. The test strip of the present invention was superior to the control kit in terms of the storage temperature.

With respect to clinical relevance: the type of the test sample for the test strip of the present invention might be serum, plasma and whole blood, and the type of the test sample for the control reagent did not include the whole blood. The whole blood could not be directly loaded onto a machine due to the presence of blood cells. Therefore, the type of the control sample was serum, and the detection result of the whole blood sample was shown in Embodiment 6. The control reagent was a digoxin determination kit (legal luminescent microparticle immunoassay) produced by Abbott Laboratories. For the results, see Table 4 and FIG. 2.

**Table 4 Test reagent and control reagent of the present invention**

| Sample No. | Test reagent | Control reagent |
|---|---|---|
| 1 | 0.22 | 0.25 |
| 2 | 0.35 | 0.37 |
| 3 | 0.67 | 0.71 |
| 4 | 0.27 | 0.29 |
| 5 | 1.35 | 1.41 |
| 6 | 0.87 | 0.91 |
| 7 | 1.49 | 1.52 |
| 8 | 2.21 | 2.36 |
| 9 | 1.33 | 1.28 |
| 10 | 2.53 | 2.36 |
| 11 | 2.42 | 2.31 |
| 12 | 5.36 | 5.28 |
| 13 | 4.61 | 4.83 |
| 14 | 0.56 | 0.53 |
| 15 | 0.89 | 0.93 |
| 16 | 1.46 | 1.53 |
| 17 | 0.53 | 0.46 |
| 18 | 0.86 | 0.95 |
| 19 | 1.53 | 1.62 |
| 20 | 2.01 | 2.11 |
| 21 | 4.53 | 4.37 |
| 22 | 3.24 | 3.41 |
| 23 | 3.81 | 3.92 |
| 24 | 0.91 | 0.87 |
| 25 | 1.21 | 1.25 |
| 26 | 3.01 | 3.08 |
| 27 | 3.11 | 3.13 |
| 28 | 4.19 | 4.21 |
| 29 | 3.24 | 3.56 |
| 30 | 2.79 | 2.98 |

As can be seen from Table 4, the clinical relevance is greater than 0.9, which is in line with clinical use requirements.

It can be seen from the results of the above Embodiments that, the immunochromatographic test strip provided by the present invention has good repeatability and an anti-interference property within an acceptable range, can be used for detecting the whole blood sample, and has excellent stability.

The foregoing descriptions are only preferred implementation manners of the present invention.

## Claims

1. An immunochromatographic test strip for detecting digoxin, comprising a bottom plate, and a sample pad, a glass fiber mat, a nitrocellulose membrane and a water absorbing paper which are sequentially overlapped on the bottom plate, wherein the glass fiber mat is sprayed with a digoxin-specific antibody-fluorescent microsphere complex; and the nitrocellulose membrane is sequentially provided with a detection line coated with digoxin protein conjugate and a quality control line coated with a secondary antibody;
the digoxin-specific antibody-fluorescent microsphere complex is obtained by conjugation between a digoxin monoclonal antibody or a digoxin polyclonal antibody and a fluorescent microsphere; the mass percentage concentration of the digoxin-specific antibody-fluorescent microsphere complex is 0.01-0.1%; and the spraying amount of the digoxin-specific antibody-fluorescent microsphere complex on the glass fiber mat is 1-10 µL/cm; and
the digoxin protein conjugate comprises a digoxin-conjugated rabbit albumin or a digoxin-conjugated rabbit ovalbumin; the concentration of the digoxin-conjugated rabbit albumin is 1 mg/mL; the concentration of the digoxin-conjugated rabbit ovalbumin is 3 mg/mL; and the spraying amount of the digoxin protein conjugate on the nitrocellulose membrane is 1-5 µL/cm; and
the secondary antibody comprises a goat anti-mouse IgG antibody; and
the sample pad comprises a glass fiber, a polyester film, a cellulose filter paper, and a nonwoven fabric.

2. Use of the immunochromatographic test strip according to claim 1 for detecting digoxin content in a sample.

3. The use according to claim 2, wherein the detecting the digoxin content in the sample comprises the steps of:
1) adding a sample to be tested dropwise onto a sample pad for chromatography;
2) reading fluorescence signal values, i.e., a T value and a C value, for the detection line and the quality control line of the immunochromatographic test strip after the chromatography; and
3) substituting the fluorescence signal values T/C into a standard linear regression equation *y* = *ax*³ + *bx*² + *cx* + *d* to calculate the digoxin content in the sample to be tested.

4. The use according to claim 2, wherein the test sample comprises serum, plasma and whole blood.

## Patentansprüche

1. Immunochromatographischer Teststreifen zum Nachweis von Digoxin, umfassend eine Bodenplatte und ein Probenkissen, eine Glasfasermatte, eine Nitrocellulosemembran und ein wasserabsorbierendes Papier, die nacheinander auf der Bodenplatte überlappend angeordnet sind, wobei die Glasfasermatte mit einem Digoxin-spezifischen Antikörper-fluoreszierenden Mikrokügelchen-Komplex besprüht ist; und die Nitrocellulosemembran nacheinander mit einer Nachweislinie, die mit einem Digoxin-Protein-Konjugat beschichtet ist, und einer Qualitätskontrolllinie, die mit einem sekundären Antikörper beschichtet ist, versehen ist;
der Digoxin-spezifische Antikörper-fluoreszierende Mikrokügelchen-Komplex durch Konjugation zwischen einem monoklonalen Digoxin-Antikörper oder einem polyklonalen Digoxin-Antikörper und einem fluoreszierenden Mikrokügelchen erhalten wird; die prozentuale Massenkonzentration des Digoxin-spezifischen Antikörper-fluoreszierenden Mikrokügelchen-Komplexes 0,01-0,1 % beträgt; und die Sprühmenge des Digoxin-spezifischen Antikörper-fluoreszierenden Mikrokügelchen-Komplexes auf der Glasfasermatte 1-10 µl/cm beträgt; und
das Digoxin-Protein-Konjugat ein Digoxin-konjugiertes Kaninchenalbumin oder ein Digoxin-konjugiertes Kaninchenovalbumin umfasst; die Konzentration des Digoxinkonjugierten Kaninchenalbumins 1 mg/ml beträgt; die Konzentration des Digoxinkonjugierten Kaninchenovalbumins 3 mg/ml beträgt; und die Sprühmenge des Digoxin-Protein-Konjugats auf die Nitrocellulosemembran 1-5 µl/cm beträgt; und
der sekundäre Antikörper einen Ziegen-Anti-Maus-IgG-Antikörper umfasst; und
das Probenkissen eine Glasfaser, eine Polyesterfolie, ein Zellulosefilterpapier und einen Vliesstoff umfasst.

2. Verwendung des immunochromatographischen Teststreifens nach Anspruch 1 zum Nachweis des Digoxingehalts in einer Probe.

3. Verwendung nach Anspruch 2, wobei der Nachweis des Digoxingehalts in der Probe die folgenden Schritte umfasst:
1) tropfenweises Zugeben einer zu untersuchenden Probe auf ein Probenkissen für die Chromatographie;
2) Ablesen von Fluoreszenzsignalwerten, d. h. eines T-Wertes und eines C-Wertes, für die Nachweislinie und die Qualitätskontrolllinie des immunchromatographischen Teststreifens nach der Chromatographie; und
3) Einsetzen der Fluoreszenzsignalwerte TIC in eine lineare Standardregressionsgleichung *y* = *ax*³ + *bx*² + *cx* + *d* zur Berechnung des Digoxingehalts in der zu untersuchenden Probe.

4. Verwendung nach Anspruch 2, wobei die Testprobe Serum, Plasma und Vollblut umfasst.

## Revendications

1. Bandelette d'essai immunochromatographique pour détecter la digoxine, comprenant une plaque de fond et un tampon d'échantillon, un tapis de fibres de verre, une membrane de nitrocellulose et un papier absorbant l'eau qui sont superposés séquentiellement sur la plaque de fond, dans laquelle le tapis de fibres de verre est pulvérisé avec un complexe anticorps spécifique de la digoxine-microsphère fluorescente ; et la membrane de nitrocellulose est munie séquentiellement d'une ligne de détection enduite de conjugué de protéine de digoxine et d'une ligne de contrôle de qualité enduite d'un anticorps secondaire;
le complexe anticorps spécifique de la digoxine-microsphère fluorescente est obtenu par conjugaison entre un anticorps monoclonal de digoxine ou un anticorps polyclonal de digoxine et une microsphère fluorescente ; la concentration en pourcentage de masse du complexe anticorps spécifique de la digoxine-microsphère fluorescente est de 0,01 à 0,1 %; et la quantité de pulvérisation du complexe anticorps spécifique de la digoxine-microsphère fluorescente sur le tapis de fibres de verre est de 1 à 10 µL/cm; et
le conjugué de protéines de digoxine comprend une albumine de lapin conjuguée à la digoxine ou une ovalbumine de lapin conjuguée à la digoxine; la concentration de l'albumine de lapin conjuguée à la digoxine est de 1 mg/mL; la concentration de l'ovalbumine de lapin conjuguée à la digoxine est de 3 mg/mL; et la quantité de pulvérisation du conjugué de protéines de digoxine sur la membrane de nitrocellulose est de 1 à 5 µL/cm; et
l'anticorps secondaire comprend un anticorps IgG anti-souris de chèvre; et
le tampon d'échantillon comprend une fibre de verre, un film de polyester, un papier filtre en cellulose et un tissu non tissé.

2. Utilisation de la bandelette d'essai immunochromatographique selon la revendication 1 pour détecter la teneur en digoxine dans un échantillon.

3. Utilisation selon la revendication 2, dans laquelle la détection de la teneur en digoxine dans l'échantillon comprend les étapes suivantes:
1) ajout d'un échantillon à tester goutte à goutte sur un tampon d'échantillon pour la chromatographie;
2) lecture des valeurs du signal de fluorescence, c'est-à-dire une valeur T et une valeur C, pour la ligne de détection et la ligne de contrôle de qualité de la bandelette d'essai immunochromatographique après la chromatographie; et
3) remplacer les valeurs du signal de fluorescence TIC à une équation de régression linéaire standard *y* = *ax*³ + *bx*² + *ex* + *d* pour calculer la teneur en digoxine de l'échantillon à tester.

4. Utilisation selon la revendication 2, dans laquelle l'échantillon d'essai comprend du sérum, du plasma et du sang total.
